# EUROPEAN PATENT SPECIFICATION

(11) **EP 4 493 396 B1**
(45) Date of publication and mention of the grant of the patent: **17.12.2025**
(21) Application number: 22790080.0
(22) Date of filing: 29.09.2022
(51) Int. Cl.: B29C 65/20, B29C 65/22, B29C 65/30, B29C 65/78, A61M 39/14, H05B 3/26, A61M 39/08, B29L 23/00, H05B 3/00

(54) **FUSION BONDING WAFER, TUBE FUSION BONDING DEVICE, AND TUBE FUSION BONDING METHOD**
FUSIONSBONDWAFER, ROHRFUSIONSBONDVORRICHTUNG UND ROHRFUSIONSBONDVERFAHREN
PLAQUETTE DE LIAISON PAR FUSION, DISPOSITIF DE LIAISON DE TUBES PAR FUSION ET PROCÉDÉ DE LIAISON DE TUBES PAR FUSION

(43) Date of publication of application: 22.01.2025
(73) Proprietor: Terumo Kabushiki Kaisha, Tokyo 151-0072 (JP); Terumo BCT, Inc., Lakewood, CO 80215 (US)
(72) Inventor: PAEPS, Filip, 3001 Leuven (BE); ROELANDT, Maarten, 3001 Leuven (BE); BORTELS, Joachim, 3001 Leuven (BE); VANDEKERCKHOVE, Lucas, 3001 Leuven (BE)
(74) Representative: TBK
(86) International application number: PCT/JP2022/036370
(87) International publication number: WO 2024/069835

(56) References cited:
- EP-A1- 0 139 350
- EP-A1- 3 907 060
- US-A- 4 864 101

## Description

### [Technical Field]

The present invention relates to a fusion bonding wafer, a tube fusion bonding device, and a tube fusion bonding method configured to connect resin tubes by heating and fusion bonding them together.

### [Background Art]

Conventionally, in order to aseptically connect resin tubes (hereinafter, referred to as tubes) such as tubes for medicinal use or the like, a tube fusion bonding device is used that connects the tubes by melting and fusion bonding them together. Such a tube fusion bonding device is disclosed, for example, in JP 3220229 B2**.**

The above-described tube fusion bonding device includes a pair of clamps by which two tubes to be connected are retained, and a fusion bonding wafer. The heated fusion bonding wafer is inserted between the pair of clamps retaining the two tubes so as to intersect the tubes, and thereby cuts the two tubes. Next, the clamps are made to move so that the tubes to be connected are placed face-to-face with each other with the fusion bonding wafer being interposed therebetween. Then, the fusion bonding wafer is pulled out, and the two tubes that face toward each other are placed in direct contact to thereby fusion bond the two tubes. The fusion bonding wafer is equipped with a heating element which is sandwiched between plate-shaped substrates, and the heating element serves to heat the fusion bonding wafer to a predetermined temperature required for fusion bonding.

EP 0 139 350 A1 discloses a heating element for welding thermoplastic tubes together. The heating element comprises a folded sheet and a resistor that is disposed in a fold of the folded sheet. An opening is provided for a signal means that is a wire connected to the resistor.

US 4 864 101 A discloses a thin wafer for cutting plastic tubes which are to be butt welded together. The wafer comprises a core of metallic material to which a resistance circuit is provided and that is covered by a glass layer that can be in contact with the tube to be handled. On the glass layer a metal cladding is provided. An open area is provided in the metal cladding at an area which contacts and cuts through the tube.

EP 3 907 060 A1 discloses a multi-purpose heating blade capable of carrying out several heat-sealing operations on a tube, comprising a sheet and a heating device being resistive which is capable of increasing the temperature of the sheet to a predetermined temperature, the sheet comprising an external face intended to come into contact with a tube, the external face being provided with a non-stick coating.

### [Summary of Invention]

However, a problem arises in that, in such a conventional fusion bonding wafer as in JP 3220229 B2, it is difficult to accurately detect the temperature.

Thus, the present invention has the object of providing a fusion bonding wafer, a tube fusion bonding device, and a tube fusion bonding method that are capable of accurately detecting the temperature.

The above object is solved by a fusion bonding wafer having the features of claim 1.

A tube fusion bonding device is stated in claim 6.

A tube fusion bonding method is stated in claim 9. Further developments are subject-matter of the dependent claims.

According to the fusion bonding wafer, the tube fusion bonding device, and the tube fusion bonding method having the above-described aspects, the temperature of the fusion bonding wafer can be accurately detected.

### [Brief Description of Drawings]

[FIG. 1]

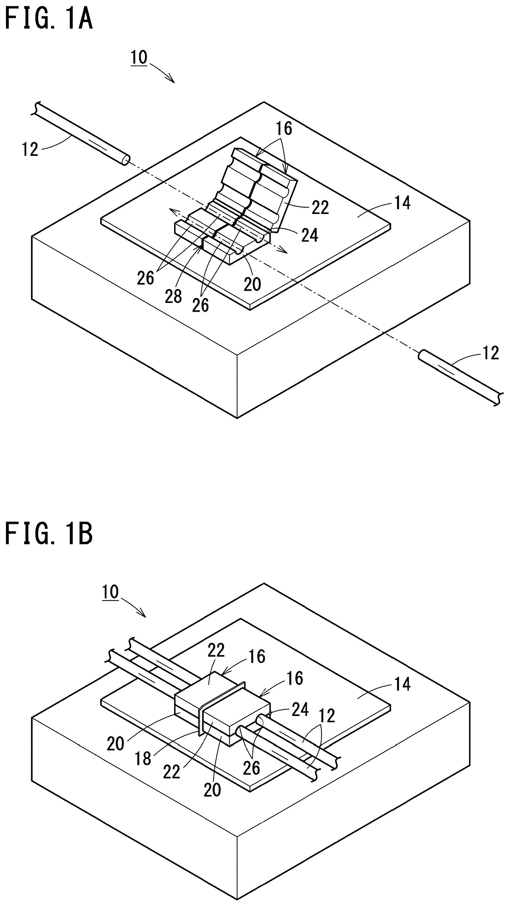

   FIG. 1A is a perspective view of a tube fusion bonding device according to an embodiment, and FIG. 1B is a perspective view of the tube fusion bonding device of FIG. 1A in which a fusion bonding wafer is made to project out therefrom;
[FIG. 2]

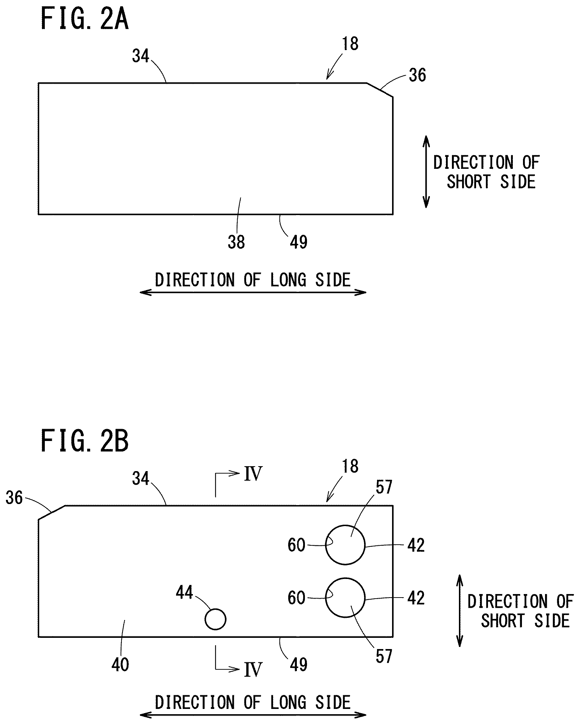

   FIG. 2A is a front view of the fusion bonding wafer shown in FIG. 1B, and FIG. 2B is a rear view of the fusion bonding wafer shown in FIG. 1B;
[FIG. 3]

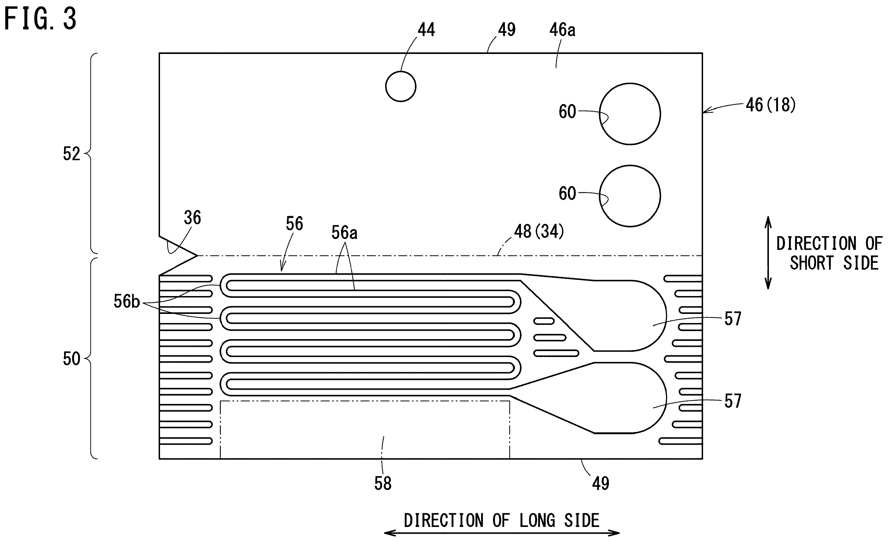

   FIG. 3 is a plan view showing a state in which the fusion bonding wafer of FIG. 2 is in an unfolded state;
[FIG. 4]

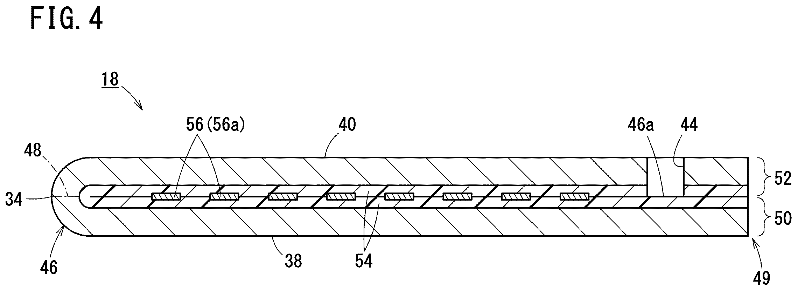

   FIG. 4 is a cross-sectional view taken along line IV-IV of FIG. 2B;
[FIG. 5]

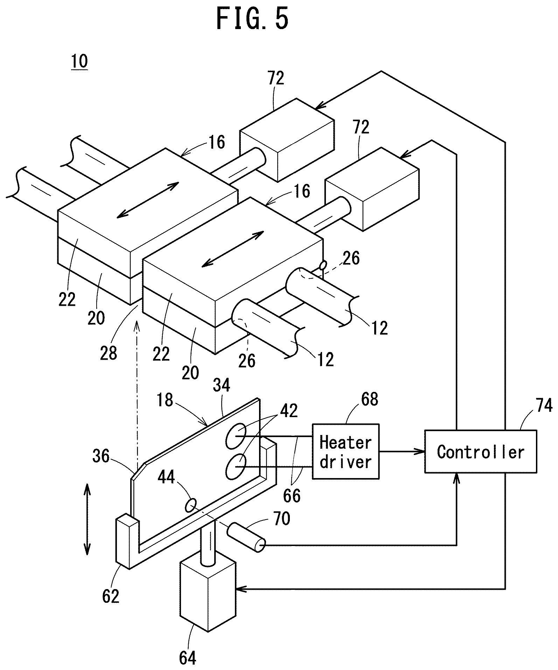

   FIG. 5 is an explanatory diagram showing a configuration of principal components of the tube fusion bonding device shown in FIG. 1;
[FIG. 6]

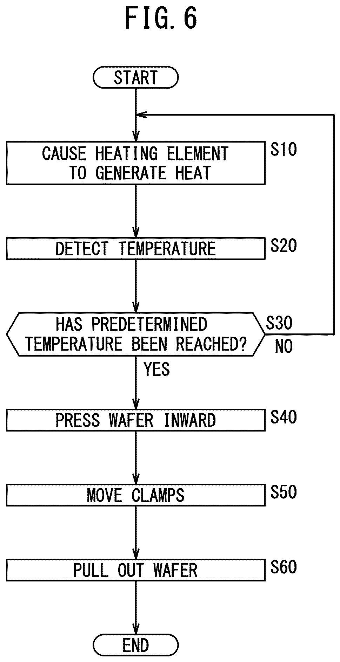

   FIG. 6 is a flowchart showing a tube fusion bonding method in which the tube fusion bonding device shown in FIG. 1 and FIG. 5 is used;
[FIG. 7]

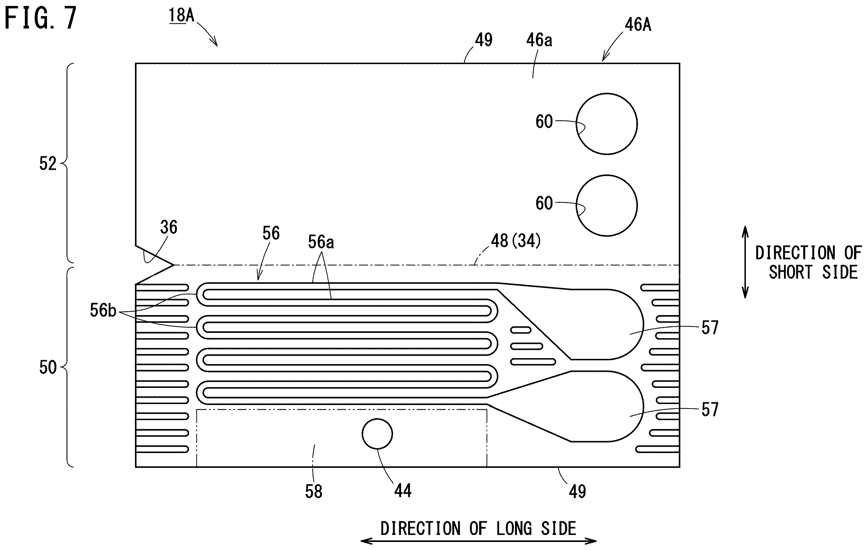

   FIG. 7 is a plan view showing an unfolded state of a fusion bonding wafer according to a second embodiment;
[FIG. 8]

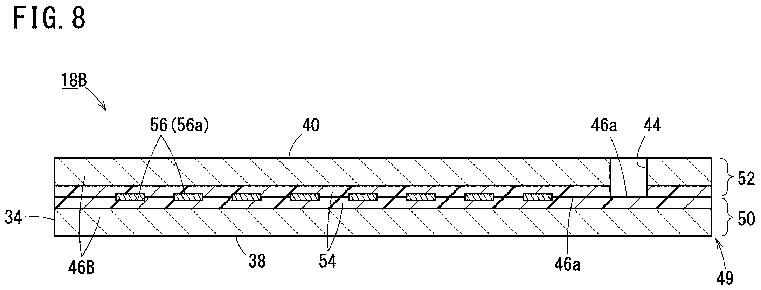

   FIG. 8 is a cross-sectional view of a fusion bonding wafer according to a third embodiment; and
[FIG. 9]

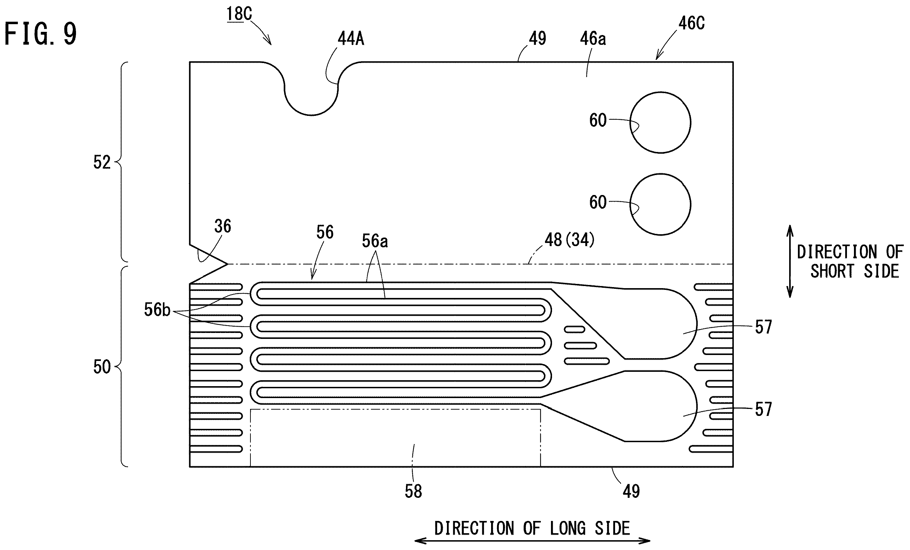

   FIG. 9 is a plan view showing an unfolded state of a fusion bonding wafer according to a fourth embodiment.

### [Description of Embodiments]

Hereinafter, a fusion bonding wafer (welding wafer) 18, a tube fusion bonding device (tubing welder) 10, and a tube fusion bonding method (tubing welding method) will be presented and described in detail below with reference to the accompanying drawings.

As shown in FIG. 1A, the tube fusion bonding device 10 according to the present embodiment includes a tube placement section 14 on which resin tubes 12 to be fusion bonded are placed. The tube placement section 14 is provided with two clamps 16 that serve to retain the resin tubes 12, and the fusion bonding wafer 18 shown in FIG. 1A.

As shown in a partially enlarged view, each of the clamps 16 is provided with a support member 20 and an arm member 22, which are vertically separated from each other. The arm member 22 is rotatably connected to the support member 20 via a hinge 24. Two grooves 26 that serve to retain the resin tubes 12 in parallel are provided on the upper surface of the support member 20. The two clamps 16 are arranged alongside one another in parallel in a longitudinal direction. A narrow groove-shaped gap 28 is disposed between the two clamps 16, and the fusion bonding wafer 18, which is not illustrated in FIG. 1, is mounted in such a gap 28 in an exchangeable manner.

Although the method of use thereof is not particularly limited, in the tube fusion bonding device 10, normally, the fusion bonding wafer 18 is a disposable component that is replaced each time that fusion bonding is performed.

As shown in FIG. 2A, each of the fusion bonding wafers 18 is formed in a long rectangular shape, and one long side thereof serves as an incision side 34 that cuts into the resin tubes 12. In a corner adjacent to the incision side 34, a notch (cutout portion) 36 is provided in order to indicate a position of the incision side 34, and serves in order to identify the mounting direction of the fusion bonding wafer 18 in the tube fusion bonding device 10.

A front surface 38 of the fusion bonding wafer 18 is constituted only by a smooth plane. On the other hand, as shown in FIG. 2B, electrode portions 42 and a temperature measurement hole 44 are formed on a rear surface 40 of the fusion bonding wafer 18. The electrode portions 42 are connected to connection terminals of the tube fusion bonding device 10, and an electrical current for heating is supplied to a wiring pattern 56, which will be described later. The temperature measurement hole 44 is used to detect the temperature in the interior of the fusion bonding wafer 18.

The fusion bonding wafer 18 is formed by folding one thin metal plate 46 shown in FIG. 3. The thin metal plate 46, for example, is made up from a metal such as copper, a copper alloy, or stainless steel or the like, and is formed in a rectangular shape wherein a dimension in a direction of the long side is on the order of 80 mm, and a dimension in a direction of the short side is on the order of 20 mm. A thickness of the thin metal plate 46, for example, is on the order of 0.5 mm.

The thin metal plate 46 is folded back on itself in an overlapping manner along a folding line 48 passing through the midpoints of the short sides to thereby form the fusion bonding wafer 18. As shown in FIG. 3, a portion on a lower side of the folding line 48 is a first substrate 50, and a portion on an upper side of the folding line 48 is a second substrate 52. More specifically, the first substrate 50 and the second substrate 52 are integrally connected together through the folding line 48.

The surface that appears in FIG. 3 is an inner surface 46a, which is a surface that does not appear on the outer side of the completed fusion bonding wafer 18. As shown in FIG. 4, the inner surface 46a of the thin metal plate 46 is covered with an insulating layer 54 made of a resin or the like. The insulating layer 54 is made up, for example, from a thermoplastic resin such as an acrylic resin or the like, and functions as an adhesive that joins the first substrate 50 and the second substrate 52 together by overlapping them in a heated state.

As shown in FIG. 3, in the inner surface 46a in an unfolded state, the wiring pattern 56 that constitutes the heating element is formed on the first substrate 50. The wiring pattern 56 is formed by solidifying a powder of a conductive material such as silver or the like with a binder or the like, and possesses conductivity. The wiring pattern 56 is formed on the insulating layer 54. The wiring pattern 56 comprises a plurality of linear portions 56a that extend in the direction of the long side, and curved portions 56b connecting the linear portions 56a. The wiring pattern 56 constitutes one meandering shape in which the linear portions 56a are connected by the curved portions 56b in the form of a U-turn. The shape of the wiring pattern 56 is not limited to a meandering shape, and various variations such as a plate-shaped pattern or the like can be adopted therefor.

In the first substrate 50, the wiring pattern 56 is provided as a pattern that causes a uniform heat generation density to be generated in the vicinity of the incision side 34 (the folding line 48), which forms a side that cuts into the resin tubes 12 that serve as objects to be connected. The wiring pattern 56 is separated away from an opposing side 49 that faces toward the folding line 48. A blank region 58 in which the wiring pattern 56 is not formed is formed between the opposing side 49 and the wiring pattern 56. Further, connection pads 57 are provided respectively at one end and the other end of the wiring pattern 56. The connection pads 57 are formed to be wider than the linear portions 56a. The connection pads 57 are provided in close proximity to the short side on a side where the cutout portion 36 is not formed, and are arranged alongside one another in the direction of the short side.

One pair of contact holes 60 and the temperature measurement hole 44 are formed in portions of the second substrate 52. The contact holes 60 and the temperature measurement hole 44 are holes which are formed to penetrate through the second substrate 52 in the thickness direction, and for example, are formed to be circular as shown in the drawing. When the second substrate 52 is overlapped on the first substrate 50, the contact holes are formed in portions corresponding to the connection pads 57. As shown in FIG. 4, in a state in which the second substrate 52 is superimposed thereon, the connection pads 57 are exposed respectively at bottom parts of the contact holes 60. The electrode portions 42 are constituted by the connection pads 57 and the contact holes 60.

As shown in FIG. 3, the temperature measurement hole 44 is disposed at a site that avoids the wiring pattern 56 of the first substrate 50 and that does not interfere with the resin tubes 12 when the fusion bonding wafer 18 is pressed into the resin tubes 12. More specifically, the temperature measurement hole 44 is provided in a portion of the second substrate 52 that corresponds to the blank region 58 in the vicinity of the opposing side 49 that faces toward the folding line 48 (the incision side 34).

As shown in FIG. 4, in a state in which the second substrate 52 is overlapped on the first substrate 50, the inner surface 46a (the insulating layer 54) of the first substrate 50 is exposed on a bottom part of the temperature measurement hole 44. From the standpoint of preventing errors in the temperature measurement result due to heat dissipation, the diameter of the temperature measurement hole 44 is preferably made as small as possible, and as shown in FIG. 3, the temperature measurement hole 44 is formed with a size smaller than that of the contact holes 60. Although not particularly limited to this feature, the diameter of the temperature measurement hole 44 can be on the order of 1 to 2 mm.

For forming the fusion bonding wafer 18 according to the present embodiment, the inner surface 46a of the thin metal plate 46 is coated with an acrylic resin or the like to thereby form the insulating layer 54 and thereafter the thin metal plate is formed into the rectangular shape shown in FIG. 3, by means of press molding. Next, the contact holes 60 and the temperature measurement hole 44 are punched out and formed. Thereafter, the wiring pattern 56 is formed on the insulating layer 54 by coating thereon an ink containing a conductive material such as silver or the like by a screen printing method or the like. Thereafter, the thin metal plate 46 is folded along the folding line 48, the second substrate 52 is overlaid on the first substrate 50, and by heating and pressing them, the first substrate 50 and the second substrate 52 are joined together via the insulating layer 54, thereby completing the fusion bonding wafer 18 having the cross-sectional structure shown in FIG. 4.

The fusion bonding wafer 18 is used by being installed in the tube fusion bonding device 10 shown in FIG. 1. As shown in FIG. 5, the tube fusion bonding device 10 is equipped with a holder 62, and the holder 62 retains the fusion bonding wafer 18 in a detachable manner. The holder 62 is supported by a wafer driving unit 64 that causes the holder 62 to move in an up-down direction (a direction across the resin tubes 12). Electrode terminals 66 that are in contact with the electrode portions 42 of the fusion bonding wafer 18 are provided in the vicinity of the holder 62. The wiring pattern 56 of the fusion bonding wafer 18 is connected to a heater driver 68 via the electrode terminals 66.

Further, the tube fusion bonding device 10 includes a temperature sensor 70, which is disposed at a position face-to-face with the temperature measurement hole 44 of the fusion bonding wafer 18. The temperature sensor 70, for example, is an infrared radiation thermometer, and detects the temperature of the inner surface 46a that is exposed through the temperature measurement hole 44 in a non-contact manner. Moreover, it should be noted that the temperature sensor 70 is not necessarily limited to being the infrared radiation thermometer, and may be a contact sensor in which a temperature measurement probe is placed in contact with the inner surface 46a.

The pair of clamps 16 are disposed on both sides of the holder 62, and the clamps 16 are supported by clamp driving units 72. The clamp driving units 72 move the clamps 16 in a direction perpendicular to the direction in which the wafer driving unit 64 moves the wafer (i.e., in the longitudinal direction of the clamps 16).

In the tube fusion bonding device 10, the wafer driving unit 64, the heater driver 68, the temperature sensor 70, and the clamp driving units 72 are connected to a controller 74. The tube fusion bonding device 10 carries out a fusion bonding operation of the resin tubes 12 under the control of a control operation of the controller 74.

Next, a description will be given concerning a tube fusion bonding method in which the fusion bonding wafer 18 and the tube fusion bonding device 10 are used.

Prior to fusion bonding the resin tubes 12, a user mounts the fusion bonding wafer 18 on the tube fusion bonding device 10 shown in FIGS. 1A and 1B. Further, the two resin tubes 12 that serve as objects to be connected are set in parallel on the clamps 16. Thereafter, when the user presses a start button of the tube fusion bonding device 10, the fusion bonding operation of the tube fusion bonding device 10 is initiated.

As shown in FIG. 6, in step S10, the tube fusion bonding device 10 causes the heating element (the wiring pattern 56) of the fusion bonding wafer 18 to generate heat. More specifically, the tube fusion bonding device 10 supplies an electrical current to the electrode portions 42 of the fusion bonding wafer 18 via the heater driver 68, and heats the fusion bonding wafer 18 by resistance heat generation of the wiring pattern 56.

Next, in step S20, the tube fusion bonding device 10 detects the temperature of the fusion bonding wafer 18. The temperature sensor 70 detects the temperature of the inner surface 46a of the fusion bonding wafer 18 through the temperature measurement hole 44 of the fusion bonding wafer 18. The heat generated in the wiring pattern 56 of the fusion bonding wafer 18 is transmitted to the surfaces of the first substrate 50 and the second substrate 52 due to thermal conduction, and the surfaces of the first substrate 50 and the second substrate 52 are heated. Since the surfaces of the first substrate 50 and the second substrate 52 dissipate heat to the external air, the temperature tends to decrease as the distance in a planar direction from the wiring pattern 56 increases, and the temperature varies significantly within the surface. In order to measure the temperature in the vicinity of the incision side 34 that cuts into the resin tubes 12, it is preferable to dispose the temperature sensor 70 in the vicinity thereof, however, such placement interferes with the resin tubes 12. For this reason, in order to prevent interference with the resin tubes 12, it is necessary to detect the temperature of the fusion bonding wafer 18 at a site that is distanced from the incision side 34.

As such, the temperature sensor 70 of the present embodiment detects the temperature in the interior of the fusion bonding wafer 18 through the temperature measurement hole 44 of the fusion bonding wafer 18. Since at the inner surface 46a of the fusion bonding wafer 18, the influence of release of heat to the exterior air is small, even at a site separated away from the incision side 34, the temperature of the inner surface 46a of the fusion bonding wafer 18 is roughly the same as the temperature in the vicinity of the incision side 34. Therefore, the temperature sensor 70 can accurately measure the temperature in the vicinity of the incision side 34.

The temperature detected by the temperature sensor 70 is input to the controller 74, and in step S30, the controller 74 determines whether or not a predetermined temperature has been reached. In this instance, the controller 74 determines whether or not the temperature of the fusion bonding wafer 18 has reached a predetermined temperature (for example, 280 degrees centigrade) that is greater than or equal to the melting point of the resin tubes 12. In step S30, in the case that the controller 74 determines that the temperature has not reached the predetermined temperature (NO), the process returns to step S20, and measurement of the temperature is continued. In step S30, in the case that the controller 74 determines that the fusion bonding wafer 18 has reached the predetermined temperature (YES), the process transitions to step S40.

In step S40, the tube fusion bonding device 10 drives the wafer driving unit 64, moves the fusion bonding wafer 18 upward, and presses the fusion bonding wafer into the two resin tubes 12 so as to cut across the two resin tubes 12. Consequently, the fusion bonding wafer 18 cuts into the resin tubes 12 from the incision side 34, and cuts the resin tubes 12 while in a state of being in close contact with the resin tubes 12.

Next, in step S50, the tube fusion bonding device 10 drives the clamp driving units 72, and causes the two resin tubes 12, which are fusion bonded target, to be placed at positions face-to-face with each other. Consequently, one of the resin tubes 12 and the other of the resin tubes 12 that serve as objects to be connected are arranged face-to-face with each other with the fusion bonding wafer 18 being interposed therebetween. Since the ends of the resin tubes 12 are in a state of being in close contact with the heated surfaces of the fusion bonding wafer 18, the ends and the interior of the resin tubes 12 are maintained in a sterile condition.

Next, in step S60, the tube fusion bonding device 10 drives the wafer driving unit 64, causes the fusion bonding wafer 18 to move downward, and pulls out the fusion bonding wafer 18 from between the pair of resin tubes 12. Owing to this feature, the melted end of one of the resin tubes 12 and the melted end of the other of the resin tubes 12 are brought into face-to-face contact with each other, and are fusion bonded together in that state. Consequently, the resin tubes 12 are fusion bonded while the interior of the resin tubes 12 is maintained in a sterile condition.

The fusion bonding wafer 18, the tube fusion bonding device 10, and the tube fusion bonding method of the present embodiment realize the following advantageous effects.

The fusion bonding wafer 18 of the present embodiment is characterized by the fusion bonding wafer 18 that fusion bonds the two resin tubes 12 together, including the first substrate 50 which is formed in a flat plate shape, the second substrate 52 joined to the inner surface 46a of the first substrate 50, the heating element **(e.g.,** the wiring pattern 56) disposed between the inner surface 46a of the first substrate 50 and the inner surface 46a of the second substrate 52, and the temperature measurement hole 44 that penetrates through the second substrate 52 in the thickness direction and is configured to expose the inner surface 46a of the first substrate 50.

According to the above-described fusion bonding wafer 18, since the temperature of the inner surface 46a of the interior of the fusion bonding wafer 18 can be detected, even in the case that the position of the temperature measurement hole 44 is located at a position separated away from the heating element, the temperature of the surface of the fusion bonding wafer 18 in the vicinity of the heating element can be measured comparatively accurately.

In the above-described fusion bonding wafer 18, the temperature measurement hole 44 may be disposed at a site that avoids the heating element. In accordance with such a configuration, since measurement of the temperature can be carried out at a position where the influence of heat generated by the heating element is avoided, an error in measuring the temperature of the fusion bonding wafer 18 can be suppressed.

In the above-described fusion bonding wafer 18, the first substrate 50 and the second substrate 52 may each include the incision side 34 that cuts into the resin tubes 12 that serve as objects to be fusion bonded, and the opposing side 49 formed on an opposite side from the incision side 34, and further, the temperature measurement hole 44 may be disposed in the vicinity of the opposing side 49. In accordance with such a configuration, the temperature measurement hole 44 can be provided at a position that does not interfere with the resin tubes 12.

In the above-described fusion bonding wafer 18, the first substrate 50 and the second substrate 52 may be formed by being connected together integrally, and the second substrate 52 may be folded back toward the inner surface 46a of the first substrate 50 at the incision side 34. In accordance with such a configuration, since the first substrate 50 and the second substrate 52 can be formed simultaneously by press working, mass productivity thereof is superior, and manufacturing costs can be reduced.

In the above-described fusion bonding wafer 18, the inner surface 46a of the first substrate 50 and the inner surface 46a of the second substrate 52 may each be covered with the insulating layer 54, and the heating element may be made up from the wiring pattern 56 that is formed on the insulating layer 54 of either one of the first substrate 50 or the second substrate 52. In accordance with such a configuration, since the wiring pattern 56 can be formed by a printing method, the production cost of the fusion bonding wafer 18 can be suppressed.

In the above-described fusion bonding wafer 18, the wiring pattern 56 may include the connection pads 57 on one end and another end thereof, and the contact holes 60 by which the connection pads 57 are exposed may be formed in one or both of the first substrate 50 and the second substrate 52. In accordance with such a fusion bonding wafer 18, electrical current can be made to flow to the wiring pattern 56 through the contact holes 60.

In the above-described fusion bonding wafer 18, the heating element (the wiring pattern 56) may be formed on the insulating layer 54 of the first substrate 50.

The tube fusion bonding device 10 of the present embodiment is configured to cut the two resin tubes 12 by the fusion bonding wafer 18 and fusion bond the two resin tubes 12 together. The tube fusion bonding device 10 includes the pair of clamps 16 that retain the two resin tubes 12, the fusion bonding wafer 18 provided to be capable of being inserted between the pair of clamps 16, the temperature sensor 70 that detects the temperature of the fusion bonding wafer 18, the clamp driving unit 72 which causes at least one of the pair of clamps 16 to be moved, the wafer driving unit 64 that causes the fusion bonding wafer 18 to be moved, and the controller 74 that controls operations of the clamp driving unit 72 and the wafer driving unit 64. The fusion bonding wafer 18 includes the first substrate 50 formed in a flat plate shape, the second substrate 52 joined to the inner surface 46a of the first substrate 50, the heating element disposed between the inner surface 46a of the first substrate 50 and the inner surface 46a of the second substrate 52, and the temperature measurement hole 44 that penetrates through the second substrate 52 in the thickness direction and exposes the inner surface 46a of the first substrate 50. The temperature sensor 70 detects the temperature of the inner surface 46a of the first substrate 50 through the temperature measurement hole 44.

According to the above-described tube fusion bonding device 10, the temperature of the fusion bonding wafer 18 can be accurately measured, even in the case that the temperature sensor 70 is arranged at a position that does not interfere with the resin tubes 12.

In the above-described tube fusion bonding device 10, the controller 74 may control operation of the wafer driving unit 64 in a manner so as to press the fusion bonding wafer 18 into the two resin tubes 12 retained by the pair of clamps 16, after the temperature of the inner surface 46a of the first substrate 50 as detected by the temperature sensor 70 has reached the predetermined temperature. In accordance with such a configuration, since a rise in temperature of the fusion bonding wafer 18 can be detected quickly, a waiting time for raising the temperature of the fusion bonding wafer 18 can be reduced, and the two resin tubes 12 can be fusion bonded together more quickly.

In the above-described tube fusion bonding device 10, the temperature sensor 70 may be a non-contact type of temperature sensor. In accordance with such a configuration, deterioration of the temperature sensor 70 can be prevented, even in the case that replacement of the fusion bonding wafer 18 is frequently performed.

The above-described tube fusion bonding method is a tube fusion bonding method for fusion bonding the two resin tubes 12 together using the fusion bonding wafer 18, wherein the fusion bonding wafer 18 includes the first substrate 50 formed in a flat plate shape, the second substrate 52 joined to the inner surface 46a of the first substrate 50, the heating element disposed between the inner surface 46a of the first substrate 50 and the inner surface 46a of the second substrate 52, and the temperature measurement hole 44 that penetrates through the second substrate 52 in the thickness direction and exposes the inner surface 46a of the first substrate 50, the fusion bonding method including the steps of retaining the two resin tubes 12 by the pair of clamps 16, detecting the temperature of the fusion bonding wafer 18 by the temperature sensor 70 through the temperature measurement hole 44, pressing the fusion bonding wafer 18, by the wafer driving unit 64, into the two resin tubes 12 retained by the pair of clamps 16 and thereby cutting the two resin tubes 12, after the temperature of the inner surface 46a of the first substrate 50 as detected by the temperature sensor 70 has reached the predetermined temperature, and fusion bonding the cut two resin tubes to each other.

According to the above-described method, since the fusion bonding wafer 18 is pressed into the resin tubes 12 after the temperature sensor 70 has detected an accurate temperature of the fusion bonding wafer 18, fusion bonding of the resin tubes 12 can be carried out reliably and quickly.

### (Second Embodiment)

In a thin metal plate 46A (fusion bonding wafer 18A) shown in FIG. 7, the temperature measurement hole 44 is provided on the side of the first substrate 50, and the temperature measurement hole 44 is not provided on the side of the second substrate 52. In a state in which the first substrate 50 and the second substrate 52 are overlapped, the inner surface 46a on the side of the second substrate 52 is exposed through the temperature measurement hole 44. Since the inner surface 46a of the first substrate 50 and the inner surface 46a of the second substrate 52 are located in close proximity to each other, the inner surface 46a of the second substrate 52 exhibits similar thermal properties to those of the inner surface 46a of the first substrate 50, and enables an accurate temperature measurement to be carried out.

### (Third Embodiment)

In a fusion bonding wafer 18B of the present embodiment shown in FIG. 8, the first substrate 50 and the second substrate 52 are made up from separate members. More specifically, the first substrate 50 and the second substrate 52 are separated at the incision side 34. The first substrate 50 and the second substrate 52 of the fusion bonding wafer 18B according to the present embodiment are constituted by a thin metal plate 46B and the insulating layer 54 provided on the side of the inner surface 46a thereof, however the present invention is not limited to this feature. Instead of the thin metal plate 46B, for example, a material that is difficult to bend such as a ceramic substrate or the like may be used. In this manner, according to the fusion bonding wafer 18B of the present embodiment, it is possible to offer a wide choice of materials, and it becomes possible to utilize materials that are less expensive.

### (Fourth Embodiment)

In a thin metal plate 46C of the present embodiment shown in FIG. 9, a temperature measurement hole 44A provided in the second substrate 52 intersects the opposing side 49. The temperature measurement hole 44A is formed as a notch (cutout portion) that is opened on the side of the opposing side 49. Even with the fusion bonding wafer 18C formed by the thin metal plate 46C of the present embodiment, an effect similar to that of the fusion bonding wafer 18 described with reference to FIGS. 2 through 4 can be obtained.

## Claims

1. A fusion bonding wafer (18) configured to fusion bond two resin tubes (12) together, the fusion bonding wafer (18) comprising:
a first substrate (50) formed in a flat plate shape;
a second substrate (52) joined to an inner surface (46a) of the first substrate (50);
a heating element (56) disposed between the inner surface (46a) of the first substrate (50) and an inner surface (46a) of the second substrate (52); and
a temperature measurement hole (44) that penetrates through the second substrate (52) in a thickness direction thereof and is configured to expose the inner surface (46a) of the first substrate (50);
wherein the first substrate (50) and the second substrate (52) each include an incision side (34) configured to cut into the resin tubes (12) to be fusion bonded, and an opposing side (49) formed on an opposite side from the incision side (34); and
the temperature measurement hole (44) is disposed in a vicinity of the opposing side (49);
**characterized in that**
a portion of the temperature measurement hole (44) intersects the opposing side (49).

2. The fusion bonding wafer (18) according to claim 1, wherein the temperature measurement hole (44) is disposed at a site that avoids the heating element (56).

3. The fusion bonding wafer (18) according to claim 1 or 2, wherein the first substrate (50) and the second substrate (52) are formed by being connected together integrally, and the second substrate (52) is folded back toward the inner surface (46a) of the first substrate (50) at the incision side (34).

4. The fusion bonding wafer (18) according to any one of claims 1 to 3, wherein the inner surface (46a) of the first substrate (50) and the inner surface (46a) of the second substrate (52) are each covered with an insulating layer (54), and the heating element (56) is made up from a wiring pattern formed on the insulating layer (54) of either one of the first substrate (50) or the second substrate (52).

5. The fusion bonding wafer (18) according to claim 4, wherein the wiring pattern includes a connection pad (57) on each of one end and another end thereof, and a contact hole (60) by which the connection pad (57) is exposed is formed in one or both of the first substrate (50) and the second substrate (52).

6. A tube fusion bonding device (10) configured to cut two resin tubes (12) by a fusion bonding wafer (18) and to fusion bond the two resin tubes (12) together, the tube fusion bonding device comprising a pair of clamps (16) configured to retain the two resin tubes (12), the fusion bonding wafer (18) configured to be inserted between the pair of clamps (16), a temperature sensor (70) configured to detect a temperature of the fusion bonding wafer (18), a clamp driving unit (72) configured to cause at least one of the pair of clamps (16) to be moved, a wafer driving unit (64) configured to cause the fusion bonding wafer (18) to be moved, and a controller (74) configured to control operations of the clamp driving unit (72) and the wafer driving unit (64);
wherein the fusion bonding wafer (18) comprises:
a first substrate (50) formed in a flat plate shape;
a second substrate (52) joined to an inner surface of the first substrate (50);
a heating element (56) disposed between the inner surface of the first substrate (50) and an inner surface of the second substrate (52); and
a temperature measurement hole (44) that penetrates through the second substrate (52) in a thickness direction thereof and is configured to expose the inner surface of the first substrate (50);
wherein the temperature sensor (70) detects a temperature of the inner surface of the first substrate (50) through the temperature measurement hole (44).

7. The tube fusion bonding device according to claim 6, wherein the controller (74) controls operation of the wafer driving unit (64) in a manner so as to press the fusion bonding wafer (18) into the two resin tubes (12) retained by the pair of clamps (16), after the temperature of the inner surface (46a) of the first substrate (50) as detected by the temperature sensor (70) has reached a predetermined temperature.

8. The tube fusion bonding device according to claim 6 or 7, wherein the temperature sensor (70) is a non-contact type of temperature sensor.

9. A tube fusion bonding method for fusion bonding two resin tubes (12) together using a fusion bonding wafer (18), wherein the fusion bonding wafer (18) comprises:
a first substrate (50) formed in a flat plate shape;
a second substrate (52) joined to an inner surface of the first substrate (50);
a heating element (56) disposed between the inner surface of the first substrate (50) and an inner surface of the second substrate (52); and
a temperature measurement hole (44) that penetrates through the second substrate (52) in a thickness direction thereof and is configured to expose the inner surface of the first substrate (50);
the fusion bonding method comprising:
retaining two resin tubes (12) by a pair of clamps (16);
detecting a temperature of the fusion bonding wafer (18) by a temperature sensor (70) through the temperature measurement hole (44);
pressing the fusion bonding wafer (18), by a wafer driving unit (64), into the two resin tubes (12) retained by the pair of clamps (16) and thereby cutting the two resin tubes (12), after a temperature of the inner surface (46a) of the first substrate (50) as detected by the temperature sensor (70) has reached a predetermined temperature; and
fusion bonding the cut two resin tubes (12) to each other.

## Patentansprüche

1. Schmelzverbindungsplättchen (18), das zum Schmelzverbinden zweier Harzrohre (12) konfiguriert ist, wobei das Schmelzverbindungsplättchen (18) Folgendes umfasst:
ein erstes Substrat (50), das in Form einer flachen Platte ausgebildet ist;
ein zweites Substrat (52), das mit einer Innenfläche (46a) des ersten Substrats (50) verbunden ist;
ein Heizelement (56), das zwischen der Innenfläche (46a) des ersten Substrats (50) und einer Innenfläche (46a) des zweiten Substrats (52) angeordnet ist; und
ein Temperaturmessloch (44), das das zweite Substrat (52) in dessen Dickenrichtung durchdringt und konfiguriert ist, um die Innenfläche (46a) des ersten Substrats (50) freizulegen;
wobei das erste Substrat (50) und das zweite Substrat (52) jeweils eine Einschnittseite (34) aufweisen, die konfiguriert ist, um in die zu verschweißenden Harzrohre (12) zu schneiden, und eine gegenüberliegende Seite (49), die auf einer der Einschnittseite (34) gegenüberliegenden Seite ausgebildet ist; und
das Temperaturmessloch (44) in der Nähe der gegenüberliegenden Seite (49) angeordnet ist;
**dadurch gekennzeichnet, dass**
ein Teil des Temperaturmesslochs (44) die gegenüberliegende Seite (49) schneidet.

2. Schmelzverbindungsplättchen (18) nach Anspruch 1, wobei das Temperaturmessloch (44) an einer Stelle angeordnet ist, die das Heizelement (56) vermeidet.

3. Schmelzverbindungsplättchen (18) nach Anspruch 1 oder 2, wobei das erste Substrat (50) und das zweite Substrat (52) durch einstückiges Verbinden miteinander gebildet sind und das zweite Substrat (52) an der Einschnittseite (34) in Richtung der Innenfläche (46a) des ersten Substrats (50) zurückgefaltet ist.

4. Schmelzverbindungsplättchen (18) nach einem der Ansprüche 1 bis 3, wobei die Innenfläche (46a) des ersten Substrats (50) und die Innenfläche (46a) des zweiten Substrats (52) jeweils mit einer Isolierschicht (54) bedeckt sind und das Heizelement (56) aus einem Leitungsmuster besteht, das auf der Isolierschicht (54) entweder des ersten Substrats (50) oder des zweiten Substrats (52) ausgebildet ist.

5. Schmelzverbindungsplättchen (18) nach Anspruch 4, wobei das Leitungsmuster ein Anschlussfeld (57) an sowohl einem Ende als auch einem anderen Ende aufweist, und ein Kontaktloch (60), durch das das Anschlussfeld (57) freigelegt ist, in dem ersten Substrat (50) oder dem zweiten Substrat (52) oder beiden ausgebildet ist.

6. Rohrschmelzverbindungsvorrichtung (10), die konfiguriert ist, um zwei Kunststoffrohre (12) mit einem Schmelzverbindungsplättchen (18) zu schneiden und die beiden Kunststoffrohre (12) miteinander zu verschweißen, wobei die Rohrschmelzverbindungsvorrichtung Folgendes aufweist: ein Paar Klemmen (16), die konfiguriert sind, um die beiden Kunststoffrohre (12) zu halten, das Schmelzverbindungsplättchen (18), das konfiguriert ist, um zwischen das Paar Klemmen (16) eingeführt zu werden, einen Temperatursensor (70), der konfiguriert ist, um eine Temperatur des Schmelzverbindungsplättchens (18) zu erfassen, eine Klemmantriebseinheit (72), die konfiguriert ist, um mindestens eine der beiden Klemmen (16) zu bewegen, eine Plättchenantriebseinheit (64), die konfiguriert ist, um das Schmelzverbindungsplättchen (18) zu bewegen, und eine Steuervorrichtung (74), die konfiguriert ist, um einen Betrieb der Klemmantriebseinheit (72) und der Plättchenantriebseinheit (64) zu steuern;
wobei das Schmelzverbindungsplättchen (18) Folgendes umfasst:
ein erstes Substrat (50), das in Form einer flachen Platte ausgebildet ist;
ein zweites Substrat (52), das mit einer Innenfläche des ersten Substrats (50) verbunden ist;
ein Heizelement (56), das zwischen der Innenfläche des ersten Substrats (50) und einer Innenfläche des zweiten Substrats (52) angeordnet ist; und
ein Temperaturmessloch (44), das das zweite Substrat (52) in dessen Dickenrichtung durchdringt und konfiguriert ist, um die Innenfläche des ersten Substrats (50) freizulegen;
wobei der Temperatursensor (70) eine Temperatur der Innenfläche des ersten Substrats (50) durch das Temperaturmessloch (44) erfasst.

7. Rohrschmelzverbindungsvorrichtung nach Anspruch 6, wobei die Steuervorrichtung (74) den Betrieb der Plättchenantriebseinheit (64) steuert, so dass das Schmelzverbindungsplättchen (18) in die beiden von dem Paar Klemmen (16) gehaltenen Harzrohre (12) gedrückt wird, nachdem die vom Temperatursensor (70) erfasste Temperatur der Innenfläche (46a) des ersten Substrats (50) eine vorbestimmte Temperatur erreicht hat.

8. Rohrschmelzverbindungsvorrichtung nach Anspruch 6 oder 7, wobei der Temperatursensor (70) ein berührungsloser Temperatursensor ist.

9. Rohrschmelzverbindungsverfahren zum Schmelzverbinden von zwei Harzrohren (12) unter Verwendung eines Schmelzverbindungsplättchens (18), wobei das Schmelzverbindungsplättchen (18) Folgendes umfasst:
ein erstes Substrat (50), das in Form einer flachen Platte ausgebildet ist;
ein zweites Substrat (52), das mit einer Innenfläche des ersten Substrats (50) verbunden ist;
ein Heizelement (56), das zwischen der Innenfläche des ersten Substrats (50) und einer Innenfläche des zweiten Substrats (52) angeordnet ist; und
ein Temperaturmessloch (44), das das zweite Substrat (52) in dessen Dickenrichtung durchdringt und konfiguriert ist, um die Innenfläche des ersten Substrats (50) freizulegen;
wobei das Schmelzverbindungsverfahren folgende Schritte umfasst:
Halten von zwei Harzrohren (12) durch ein Paar Klemmen (16);
Erfassen einer Temperatur des Schmelzverbindungsplättchens (18) mittels eines Temperatursensors (70) durch das Temperaturmessloch (44);
Pressen des Schmelzverbindungsplättchens (18) durch eine Plättchenantriebseinheit (64) in die beiden durch das Paar Klemmen (16) gehaltenen Harzrohre (12) und dadurch Schneiden der beiden Harzrohre (12), nachdem eine durch den Temperatursensor (70) erfasste Temperatur der Innenfläche (46a) des ersten Substrats (50) eine vorbestimmte Temperatur erreicht hat; und
Schmelzverbinden der beiden geschnittenen Kunststoffrohre (12) miteinander.

## Revendications

1. Plaquette de liaison par fusion (18) configurée pour lier par fusion deux tubes en résine (12) ensemble, la plaquette de liaison par fusion (18) comprenant :
un premier substrat (50) formé en forme de plaque plate ;
un deuxième substrat (52) joint à une surface intérieure (46a) du premier substrat (50) ;
un élément chauffant (56) disposé entre la surface intérieure (46a) du premier substrat (50) et une surface intérieure (46a) du deuxième substrat (52) ; et
un trou de mesure de température (44) qui traverse le deuxième substrat (52) dans un sens de son épaisseur et est configuré pour exposer la surface intérieure (46a) du premier substrat (50) ;
dans lequel le premier substrat (50) et le deuxième substrat (52) comportent chacun un côté incision (34) configuré pour couper dans les tubes en résine (12) à lier par fusion, et un côté opposé (49) formé sur un côté opposé au côté incision (34) ; et
le trou de mesure de température (44) est disposé à une proximité du côté opposé (49) ;
**caractérisé en ce que**
une partie du trou de mesure de température (44) croise le côté opposé (49).

2. Plaquette de liaison par fusion (18) selon la revendication 1, dans laquelle le trou de mesure de température (44) est disposé à un emplacement qui évite l'élément chauffant (56).

3. Plaquette de liaison par fusion (18) selon la revendication 1 ou 2, dans laquelle le premier substrat (50) et le deuxième substrat (52) sont formés en étant reliés ensemble de manière intégrale, et le deuxième substrat (52) est replié vers la surface intérieure (46a) du premier substrat (50) au côté incision (34).

4. Plaquette de liaison par fusion (18) selon l'une quelconque des revendications 1 à 3, dans laquelle la surface intérieure (46a) du premier substrat (50) et la surface intérieure (46a) du deuxième substrat (52) sont chacune recouvertes d'une couche isolante (54), et l'élément chauffant (56) est constitué d'un motif de conducteurs formé sur la couche isolante (54) de l'un ou l'autre du premier substrat (50) ou du deuxième substrat (52).

5. Plaquette de liaison par fusion (18) selon la revendication 4, dans laquelle le motif de conducteurs comporte un plot de connexion (57) à chacune d'une extrémité et d'une autre extrémité de celui-ci, et un trou de contact (60) par lequel le plot de connexion (57) est exposé est formé dans le premier substrat (50) ou le deuxième substrat (52) ou dans les deux.

6. Dispositif de liaison par fusion de tubes (10) configuré pour couper deux tubes en résine (12) à l'aide d'une plaquette de liaison par fusion (18) et pour lier par fusion les deux tubes en résine (12) ensemble, le dispositif de liaison par fusion de tubes comprenant une paire de pinces (16) configurées pour retenir les deux tubes en résine (12), la plaquette de liaison par fusion (18) configurée pour être insérée entre la paire de pinces (16), un capteur de température (70) configuré pour détecter une température de la plaquette de liaison par fusion (18), une unité d'entraînement de pince (72) configurée pour provoquer le déplacement d'au moins l'une de la paire de pinces (16), une unité d'entraînement de plaquette (64) configurée pour provoquer le déplacement de la plaquette de liaison par fusion (18), et un contrôleur (74) configuré pour contrôler des opérations de l'unité d'entraînement de pince (72) et de l'unité d'entraînement de plaquette (64) ;
dans lequel la plaquette de liaison par fusion (18) comprend :
un premier substrat (50) formé en forme de plaque plate ;
un deuxième substrat (52) joint à une surface intérieure du premier substrat (50) ;
un élément chauffant (56) disposé entre la surface intérieure du premier substrat (50) et une surface intérieure du deuxième substrat (52) ; et
un trou de mesure de température (44) qui traverse le deuxième substrat (52) dans un sens de son épaisseur et est configuré pour exposer la surface intérieure du premier substrat (50) ;
dans lequel le capteur de température (70) détecte la température de la surface intérieure du premier substrat (50) à travers le trou de mesure de température (44).

7. Dispositif de liaison par fusion de tubes selon la revendication 6, dans lequel le contrôleur (74) commande le fonctionnement de l'unité d'entraînement de plaquette (64) de manière à presser la plaquette de liaison par fusion (18) dans les deux tubes en résine (12) maintenus par la paire de pinces (16), une fois que la température de la surface intérieure (46a) du premier substrat (50), telle que détectée par le capteur de température (70), a atteint une température prédéterminée.

8. Dispositif de liaison par fusion de tubes selon la revendication 6 ou 7, dans lequel le capteur de température (70) est un capteur de température de type sans contact.

9. Procédé de liaison par fusion de tubes pour lier par fusion deux tubes en résine (12) ensemble en utilisant une plaquette de liaison par fusion (18), dans lequel la plaquette de liaison par fusion (18) comprend :
un premier substrat (50) formé en forme de plaque plate ;
un deuxième substrat (52) joint à une surface intérieure du premier substrat (50) ;
un élément chauffant (56) disposé entre la surface intérieure du premier substrat (50) et une surface intérieure du deuxième substrat (52) ; et
un trou de mesure de température (44) qui traverse le deuxième substrat (52) dans un sens de son épaisseur et est configuré pour exposer la surface intérieure du premier substrat (50) ;
le procédé de liaison par fusion comprenant :
la retenue de deux tubes en résine (12) par une paire de pinces (16) ;
la détection d'une température de la plaquette de liaison par fusion (18) par un capteur de température (70) à travers le trou de mesure de température (44) ;
la pression de la plaquette de liaison par fusion (18), à l'aide d'une unité d'entraînement de plaquette (64), dans les deux tubes en résine (12) retenus par la paire de pinces (16) et couper ainsi les deux tubes en résine (12), après qu'une température de la surface intérieure (46a) du premier substrat (50), telle que détectée par le capteur de température (70), a atteint une température prédéterminée ; et
la liaison par fusion des deux tubes en résine coupés (12) l'un à l'autre.
